# FASCICULE DE BREVET EUROPEEN

(11) **EP 2 516 523 B1**
(45) Date de publication et mention de la délivrance du brevet: **15.11.2017**
(21) Numéro de dépôt: 10808926.9
(22) Date de dépôt: 22.12.2010
(51) Int. Cl.: C08J 7/16, A47K 5/12, A61M 11/00, A61M 15/00, B05D 5/08, B65D 81/28, B65D 83/14, C09D 5/14

(54) **PROCEDE DE TRAITEMENT DE SURFACE D'UN DISPOSITIF DE DISTRIBUTION DE PRODUIT FLUIDE**
VERFAHREN ZUR BEHANDLUNG DER OBERFLÄCHE EINER VORRICHTUNG ZUR AUSGABE EINES FLÜSSIGPRODUKTES
METHOD FOR TREATING THE SURFACE OF A DEVICE FOR DISPENSING A FLUID PRODUCT

(30) Priorité: 23.12.2009 FR 0959493
(43) Date de publication de la demande: 31.10.2012
(73) Titulaire: Aptar France SAS, 27110 Le Neubourg (FR)
(72) Inventeur: BRUNA, Pascal, F-76300 Sotteville Les Rouen (FR)
(74) Mandataire: CAPRI
(86) Numéro de dépôt international: PCT/FR2010/052892
(87) Numéro de publication internationale: WO 2011/077059

(56) Documents cités:
- WO-A1-01/17575
- WO-A1-02/47829
- WO-A1-03/066512
- US-A- 4 980 231
- US-A- 6 001 894
- US-A1- 2005 118 239
- US-A1- 2007 131 226
- VIEL ET AL: "Covalent grafting onto self-adhesive surface base on aryldiazonium salt seed layers", J. MATER. CHEM,, vol. 18, 5 novembre 2008 (2008-11-05), pages 5913-5920, XP002591657,
- MEVELLEC V ET AL: "Grafting polymers on surfaces: A new powerful and versatile diazonium salt-based one-step process in aqueous media", CHEM. MATER., vol. 19, 11 août 2007 (2007-08-11), pages 6323-6329, XP002562057,

## Description

La présente invention concerne un procédé de traitement de surface pour des dispositifs de distribution de produits fluides.

Les dispositifs de distribution de produits fluides sont bien connus. Ils comportent généralement un réservoir, un organe de distribution, tel qu'une pompe ou une valve, et une tête de distribution pourvue d'un orifice de distribution. En particulier dans le domaine pharmaceutique, les risques de contamination du produit fluide à distribuer peuvent être critiques, notamment lorsque ces produits sont dépourvus de conservateurs. Ainsi, des dispositifs de distribution par voie nasale ou orale peuvent subir des contaminations bactériennes. Ces contaminations peuvent se produire notamment à l'intérieur du réservoir dans lequel le produit fluide est stocké, notamment par pénétration de bactéries à travers l'orifice de distribution, ou à l'extérieur du réservoir au contact du patient, notamment autour de l'orifice de distribution. Pour limiter ces risques, il a été proposé de filtrer l'air d'éventation ou d'utiliser des pompes sans reprises d'air. On peut aussi prévoir un obturateur directement en amont de l'orifice de distribution, qui empêche la prolifération de contamination bactérienne vers le réservoir entre deux utilisations du dispositif. Ces solutions sont toutefois insuffisantes pour les surfaces externes, par exemple les parois en contact avec l'intérieur des narines lors d'une distribution nasale. Elles sont aussi insuffisantes pour les surfaces internes, notamment pendant la phase de distribution, lorsqu'un éventuel obturateur est en position ouverte. Pour davantage limiter les risques de contamination bactérienne à l'intérieur et à l'extérieur des dispositifs, il a été proposé de revêtir certaines surfaces internes et/ou externes, destinées à venir en contact avec le produit fluide à distribuer, avec des produits bactéricides et/ou bactériostatiques, par exemple des couches contenant des ions d'argent. Les documents suivants décrivent diverses solutions de l'art antérieur: EP0473892, EP0580460, EP0831972, US6227413, EP1169241, DE2830977, US5154325, EP0644785, US5433343, EP0889757, US2007131226, WO0247829, WO03066512, WO0117575, US6001894, US2005118239, US4980231. Ces solutions ne sont toutefois pas satisfaisantes. Ainsi, l'efficacité et la durabilité de ces revêtements sont discutables, et ils ne permettent généralement pas de remplir les exigences réglementaires relatives aux tests bactériologiques pour des distributeurs de médicaments sans conservateurs.

La présente invention a pour but de proposer un procédé de traitement de surface qui ne reproduit pas les inconvénients susmentionnés.

En particulier, la présente invention a pour but de fournir un procédé de traitement de surface qui soit efficace, durable, non polluant, qui n'interagisse pas avec le produit fluide et qui soit simple à réaliser.

La présente invention a donc pour objet un procédé de traitement de surface selon la revendication 1. Des modes de réalisation avantageux sont décrits dans les revendications dépendantes.

Plus particulièrement, la présente invention prévoit d'utiliser un procédé similaire à celui décrit dans le document WO 2008/078052, qui décrit un procédé de préparation d'un film organique à la surface d'un support solide dans des conditions non électrochimiques. De manière surprenante, ce type de procédé s'est avéré adapté pour former un film mince bactéricide et/ou bactériostatique sur des surfaces destinées à venir en contact avec un produit fluide pharmaceutique dans des dispositifs de distribution du type nasal ou oral. Une telle application de ce procédé de greffage n'avait jamais été envisagée.

De manière synthétique, le procédé a pour but de préparer un film mince à la surface d'un support solide, notamment en polyéthylène et/ou en polypropylène. Ce procédé comprend principalement une mise en contact de ladite surface de support avec une solution liquide. Celle-ci comprend au moins un solvant et au moins un primaire d'adhésion permettant la formation d'entités radicalaires à partir du primaire d'adhésion.

Le "film mince" peut être tout film bactéricide et/ou bactériostatique, notamment de nature organique, par exemple issu de plusieurs unités d'espèces chimiques organiques, et lié de manière covalente à la surface du support sur lequel est effectué le procédé. Il s'agit particulièrement d'un film lié de manière covalente à la surface du support et comprenant au moins une couche d'unités structurales de nature similaires. Selon l'épaisseur du film, sa cohésion est assurée par les liaisons covalentes qui se développent entre les différentes unités. De préférence, le film mince contient des ions d'argent.

Le solvant employé dans le cadre du procédé peut être de nature protique ou aprotique. Il est préférable que le primaire soit soluble dans ledit solvant.

Par "solvant protique" on entend un solvant qui comporte au moins un atome d'hydrogène susceptible d'être libéré sous forme de proton. Le solvant protique peut être choisi dans le groupe constitué par l'eau, l'eau désionisée, l'eau distillée, acidifiées ou non, l'acide acétique, les solvants hydroxylés comme le méthanol et l'éthanol, les glycols liquides de faible poids moléculaire tels que l'éthylèneglycol, et leurs mélanges. Dans une première variante, le solvant protique n'est constitué que par un solvant protique ou par un mélange de différents solvants protiques. Dans une autre variante, le solvant protique ou le mélange de solvants protiques peut être utilisé en mélange avec au moins un solvant aprotique, étant entendu que le mélange résultant présente les caractéristiques d'un solvant protique. L'eau acidifiée est le solvant protique préféré et, plus particulièrement, l'eau distillée acidifiée ou l'eau désionisée acidifiée.

Par "solvant aprotique" on entend un solvant qui n'est pas considéré comme protique. De tels solvants ne sont pas susceptibles de libérer un proton ou d'en accepter un dans des conditions non extrêmes. Le solvant aprotique est avantageusement choisi parmi la diméthylformamide (DMF), l'acétone et le diméthyl sulfoxyde (DMSO).

Le terme "primaire d'adhésion" correspond à toute molécule organique susceptible, sous certaines conditions, de se chimisorber à la surface du support solide par réaction radicalaire tel qu'un greffage chimique radicalaire. De telles molécules comportent au moins un groupe fonctionnel susceptible de réagir avec un radical et également une fonction réactive vis-à-vis d'un autre radical après chimisorption. Ces molécules sont ainsi capables de former un film de nature polymérique après greffage d'une première molécule à la surface du support puis réaction avec d'autres molécules présentes dans son environnement.

Le terme "greffage chimique radicalaire" se réfère notamment à l'utilisation d'entités moléculaires possédant un électron non apparié pour former des liaisons de type liaison covalente avec la surface de support, lesdites entités moléculaires étant générées indépendamment de la surface de support sur laquelle elles sont destinées à être greffées. Ainsi, la réaction radicalaire conduit à la formation de liaisons covalentes entre la surface de support concernée et le dérivé du primaire d'adhésion greffé puis entre un dérivé greffé et des molécules présentes dans son environnement.

Par "dérivé du primaire d'adhésion" on entend une unité chimique résultant du primaire d'adhésion, après que ce dernier a réagi par greffage chimique radicalaire notamment avec la surface du support solide, ou avec un autre radical. Il est clair pour l'homme du métier que la fonction réactive vis-à-vis d'un autre radical après chimisorption du dérivé du primaire d'adhésion est différente de la fonction impliquée dans la liaison covalente notamment avec la surface du support solide. Le primaire d'adhésion est avantageusement un sel d'aryle clivable choisi dans le groupe constitué par les sels d'aryle diazonium, les sels d'aryle d'ammonium, les sels d'aryle phosphonium et les sels d'aryle sulfonium.

En variante aux liaisons covalentes directes des ions d'argent sur la surface de support, obtenues en milieu aqueux, on peut aussi utiliser un procédé d'imprégnation d'une couche poreuse préalablement greffée avec des ions d'argent.

Avantageusement, le traitement de surface anti-bactérien de la présente invention peut être combiné avec un ou plusieurs autres traitements de surfaces pour donner une ou plusieurs autres propriétés à la surface de support traitée. En particulier, des films minces supplémentaires peuvent être formés par greffage chimique sur la même surface de support pour limiter les frottements, limiter le collage de produit actif et/ou empêcher l'interaction entre le produit fluide et la surface de support. Ceci peut être réalisé en plusieurs étapes de greffage successives, réalisés dans des bains mono composant spécifiques, selon un ordre quelconque. En variante, on peut envisager une seule et même étape de greffage chimique dans un bain multi composants.

Des tests ont été effectués, consistants à traiter des échantillons de matière polymérique (polypropylène) en greffant un revêtement contenant des ions métalliques. Des résultats probants ont été obtenus avec un revêtement à base de polyacide acrylique (PAA) associé à des ions d'argent, notamment des ions Ag⁰ (argent réduit) et des ions Ag⁺ (argent oxydé). Les tests ont concernés les microorganismes S.aureus et P.aeruginosa. On a constaté une absence ou diminution de prolifération sous et autour des zones traitées.

Diverses modifications sont également possibles pour un homme du métier sans sortir du cadre de la présente invention tel que défini par les revendications annexées.

## Revendications

1. Procédé de traitement de surface d'un dispositif de distribution de produit fluide comportant un réservoir contenant le produit fluide, un organe de distribution, tel qu'une pompe ou une valve fixée sur ledit réservoir, et une tête de distribution pourvue d'un orifice de distribution prévue pour actionner ledit organe de distribution, **caractérisé en ce que** ledit procédé comprend l'étape de former par greffage chimique un film mince d'épaisseur inférieure à 1 micromètre sur au moins une surface de support d'au moins une partie dudit dispositif en contact avec ledit produit fluide, ledit film mince ayant des propriétés bactéricides et/ou bactériostatiques, ledit film mince étant un film polymérique comportant des ions d'argent, ledit greffage chimique créant des liaisons covalentes entre les molécules dudit film mince et ladite surface de support, ledit greffage chimique étant réalisé dans un milieu aqueux, ladite étape de greffage chimique étant initiée par activation chimique d'un sel de diazonium pour former une couche d'ancrage pour ledit film mince.

2. Procédé selon la revendication 1, dans lequel lesdits ions d'argent sont sous forme oxydée.

3. Procédé selon la revendication 1 ou 2, dans lequel ladite surface de support est en matériau synthétique, comprenant notamment du polyéthylène et/ou du polypropylène.

4. Procédé selon la revendication 1 ou 2, dans lequel ladite surface de support est en élastomère, en verre ou en métal.

5. Procédé selon l'une quelconque des revendications précédentes, dans lequel ledit film mince a une épaisseur comprise entre 10 et 2000 angströms.

6. Procédé selon l'une quelconque des revendications précédentes, dans lequel ledit produit fluide est un produit fluide pharmaceutique destiné à être pulvérisé de manière nasale ou orale.

## Patentansprüche

1. Verfahren zur Behandlung der Oberfläche einer Vorrichtung zur Abgabe eines Flüssigprodukts, umfassend einen Behälter mit dem Flüssigprodukt, ein Abgabeelement, beispielsweise eine Pumpe oder ein Ventil, die bzw. das an dem Behälter angebracht ist, und einen mit einer Abgabeöffnung versehenen Abgabekopf, der zur Betätigung des Abgabeelements vorgesehen ist, **dadurch gekennzeichnet, dass** das Verfahren den Schritt der Bildung - durch chemisches Pfropfen - eines Dünnfilms einer Dicke unter 1 Mikrometer auf mindestens einer Auflagefläche mindestens eines Teils der Vorrichtung, die sich mit dem Flüssigprodukt in Kontakt befindet, beinhaltet, wobei der Dünnfilm bakterizide und/oder bakteriostatische Eigenschaften hat, der Dünnfilm ein Polymerfilm mit Silberionen ist, das chemische Pfropfen zu kovalenten Bindungen zwischen den Molekülen des Dünnfilms und der Auflagefläche führt, das chemische Pfropfen in wässrigem Medium durchgeführt wird, der Schritt des chemischen Pfropfens durch chemische Aktivierung eines Diazoniumsalzes zur Bildung einer Verankerungsschicht für den Dünnfilm eingeleitet wird.

2. Verfahren nach Anspruch 1, bei dem die Silberionen in oxidierter Form vorliegen.

3. Verfahren nach Anspruch 1 oder 2, bei dem die Auflagefläche aus Kunststoffmaterial besteht, welches insbesondere Polyethylen und/oder Polypropylen umfasst.

4. Verfahren nach Anspruch 1 oder 2, bei dem die Auflagefläche aus Elastomer, Glas oder Metall besteht.

5. Verfahren nach einem der vorhergehenden Ansprüche, bei dem der Dünnfilm eine Dicke im Bereich von 10 bis 2000 Angström umfasst.

6. Verfahren nach einem der vorhergehenden Ansprüche, bei dem das Flüssigprodukt ein pharmazeutisches Flüssigprodukt zum Einsprühen in die Nase oder den Mund ist.

## Claims

1. A treatment method for treating the surface of a fluid dispenser device device comprising a reservoir containing the fluid, a dispenser member, such as a pump or a valve fixed on said reservoir, and a dispenser head that is provided with a dispenser orifice that is provided for actuating said dispensing member, said method being **characterized in that** it comprises the step of using chemical grafting to form a thin film with a thickness that is less than 1 µm on at least one support surface of at least one portion of said device that is in contact with said fluid, said thin film having bactericidal and/or bacteriostatic properties, said thin film being a polymeric film that includes silver ions, said chemical grafting creating covalent bonds between the molecules of said thin film and said support surface, said chemical grafting is performed in an aqueous medium, said chemical-grafting step being initiated by chemically activating a diazonium salt so as to form an anchor layer for said thin film.

2. A method according to claim 1, wherein said silver ions are in oxidized form.

3. A method according to claim 1 or claim 2, wherein said support surface is made of synthetic material, in particular comprising polyethylene and/or polypropylene.

4. A method according to claim 1 or claim 2, wherein said support surface is made of elastomer, glass, or metal.

5. A method according to any preceding claim, wherein said thin film has a thickness lying in the range 10 Å to 2000 Å.

6. A method according to any preceding claim, wherein said fluid is a pharmaceutical for spraying in nasal or oral manner.
